# EUROPEAN PATENT SPECIFICATION

(11) **EP 1 100 829 B1**
(45) Date of publication and mention of the grant of the patent: **05.09.2007**
(21) Application number: 99944294.0
(22) Date of filing: 26.07.1999
(51) Int. Cl.: C07K 16/28, A61K 39/395

(54) **USE OF BASILIXIMAB IN THE TREATMENT OF RHEUMATOID ARTHRITIS OR SKIN DISEASES**
VERWENDUNG VON BASILIXIMAB ZUR BEHANDLUNG VON RHEUMATOIDER ARTHRITIS UND HAUTERKRANKUNGEN
BASILIXIMAB UTILISE POUR TRAITER LA POLYARTHRITE RHUMATOIDE OU LES MALADIES CUTANEES

(30) Priority: 27.07.1998 GB 9816281; 27.05.1999 GB 9912460
(43) Date of publication of application: 23.05.2001
(73) Proprietor: Novartis AG, 4056 Basel (CH); Novartis Pharma GmbH, 1230 Wien (AT); UNIVERSITY COLLEGE LONDON, London NW3 2PF (GB)
(72) Inventor: AMLOT, Peter, Lloyd, London N8 9BL (GB); SCHREIER, Max, H., CH-4054 Basel (CH)
(74) Representative: de Weerd, Petrus G.W.
(86) International application number: PCT/EP1999/005316
(87) International publication number: WO 2000/006604

(56) References cited:
- EP-A- 0 449 769
- WO-A-89/09622
- QUEEN C ET AL: "A HUMANIZED ANTIBODY THAT BINDS TO THE INTERLEUKIN 2 RECEPTOR" PROCEEDINGS OF THE NATIONAL ACADEMY OF SCIENCES OF USA,US,NATIONAL ACADEMY OF SCIENCE. WASHINGTON, vol. 86, no. 24, 1 December 1989 (1989-12-01), pages 10029-10033, XP000310534 ISSN: 0027-8424
- DATABASE CHEMABS [Online] CHEMICAL ABSTRACTS SERVICE, COLUMBUS, OHIO, US AN: 128:126854, J. KOVARIK ET AL: "Disposition of basiliximab, an interleukin-2 receptor monoclonal antibody, in recipients of mismatched cadaver renal allografts " XP002134122
- KOVARIK J ET AL: "DISPOSITION AND IMMUNODYNAMICS OF BASILIXIMAB IN LIVER ALLOGRAFT RECIPIENTS" CLINICAL PHARMACOLOGY & THERAPEUTICS,US,ST LOUIS, MO, vol. 64, no. 1, July 1998 (1998-07), pages 66-72, XP000876964

### SEQUENCE LISTING

<110> Novartis AG University College London
<120> CD25 binding molecules for use in the treatment of rheumatoid arthritis or skin diseases
<130> Simulect
<140>
   <141>
<160> 3
<170> Patent In Ver. 2.1
<210> 1
   <211> 5
   <212> PRT
   <213> murine
<400> 1
   Arg Tyr Trp Met His
   1 5
<210> 2
   <211> 17
   <212> PRT
   <213> murine
<400> 2
   Ala Ile Tyr Pro Gly Asn Ser Asp Thr Ser Tyr Asn Gln Lys Phe Glu
   1 5 10 15
   Gly
<210> 3
   <211> 8
   <212> PRT
   <213> murine
<400> 3
   Asp Tyr Gly Tyr Tyr Phe Asp Phe
   1 5

## Description

The invention is directed to the use of a CD25 binding molecule in the preparation of a medicament for the treatment of rheumatoid arthritis or inflammatory and hyperproliferative skin diseases, wherein the CD25 binding molecule is basiliximab.

Treatment of rheumatoid arthritis includes control or amelioration of the disease and/or its sequellae, e.g. symptoms, such as number of tender and swollen joints, degree of tenderness and swelling, or pain, as well as control or amelioration of aetiological components.

Treatment of inflammatory and hyperproliferative skin disease includes control or amelioration of the disease and/or its sequellae, e.g. symptoms, as well as control or amelioration of aetiological components. Treatment of e.g. psoriasis also includes suppression of further clinical relapse.

Inflammatory and hyperproliferative skin diseases include psoriasis, atopical dermatitis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and acne.

By "CD25 binding molecule" is meant any molecule capable of binding to the CD25 antigen either alone or associated with other molecules to form high affinity IL-2 receptors.

A CD25 binding molecule may comprise at least one antigen binding site comprising:
a) a first domain comprising in sequence the hypervariable regions CDR1, CDR2 and CDR3; said CDR1 having the amino acid sequence Arg-Tyr-Trp-Met-His, said CDR2 having the amino acid sequence Ala-Ile-Tyr-Pro-Gly-Asn-Ser-Asp-Thr-Ser-Tyr-Asn-Gln-Lys-Phe-Glu-Gly, and said CDR3 having the amino acid sequence Asp-Tyr-Gly-Tyr-Tyr-Phe-Asp-Phe and,
b) a second domain comprising in sequence the hypervariable regions CDR1', CDR2' and CDR3', said CDR1' having the amino acid sequence Ser-Ala-Ser-Ser-Ser-lle-Ser-Tyr-Met-Gln, said CDR2' having the amino acid sequence Asp-Thr-Ser-Lys-Leu-Ala-Ser, and said CDR3' having the amino acid sequence His-Gln-Arg-Ser-Ser-Tyr-Thr; or direct equivalents thereof.

Unless otherwise indicated, any polypeptide chain is herein described as having an amino acid sequence starting at the N-terminal extremity and ending at the C-terminal extremity.

When the antigen binding site comprises both the first and second domains, these may be located on the same polypeptide molecule or, preferably, each domain may be on a different chain, the first domain being part of an immunoglobulin heavy chain or fragment thereof and the second domain being part of an immunoglobulin light chain or fragment thereof.

Accordingly, a CD25 binding molecule may comprise at least one antigen binding site comprising either a first domain having an amino acid sequence identical or substantially identical to that shown in Seq. Id. No. 1 in EP 449,769, starting with amino acid at position 1 and ending with amino acid at position 117 or a first domain as described above and a second domain having an amino acid sequence identical or substantially identical to that shown in Seq. Id. No. 2 in EP 449,769, starting with amino acid at position 1 and ending with amino acid at position 104 in the treatment of rheumatoid arthritis or inflammatory and hyperproliferative skin diseases.

A CD25 binding molecule may be a chimeric anti-CD25 antibody which comprises at least
a) one immunoglobulin heavy chain or fragment thereof which comprises (i) a variable domain comprising in sequence the hypervariable regions CDR1, CDR2 and CDR3 and (ii) the constant part or fragment thereof of a human heavy chain; said CDR1 having the amino acid sequence Arg-Tyr-Trp-Met-His, said CDR2 having the amino acid sequence Ala-Ile-Tyr-Pro-Gly-Asn-Ser-Asp-Thr-Ser-Tyr-Asn-Gln-Lys-Phe-Glu-Gly, and said CDR3 having the amino acid sequence Asp-Tyr-Gly-Tyr-Tyr-Phe-Asp-Phe and
b) one immunoglobulin light chain or fragment thereof which comprises (i) a variable domain comprising in sequence the hypervariable regions CDR1', CDR2' and CDR3' and (ii) the constant part or fragment thereof of a human light chain; said CDR1' having the amino acid sequence Ser-Ala-Ser-Ser-Ser-Ile-Ser-Tyr-Met-Gln, said CDR2' having the amino acid sequence Asp-Thr-Ser-Lys-Leu-Ala-Ser, and said CDR3' having the amino acid sequence His-Gln-Arg-Ser-Ser-Tyr-Thr; and direct equivalents thereof.

Alternatively, a CD25 binding molecule may be a single chain binding molecule which comprises an antigen binding site comprising
a) a first domain comprising in sequence the hypervariable regions CDR1, CDR2 and CDR3, said hypervariable regions having the amino acid sequences as shown in Seq. Id. No. 1 in EP 449,769, the contents of which is herein incorporated by reference,
b) a second domain comprising in sequence the hypervariable regions CDR1', CDR2' and CDR3', said hypervariable regions having the amino acid sequences as shown in Seq. Id. No. 2 in EP 449,769, and
c) a peptide linker which is bound either to the N-terminal extremity of the first domain and to the C-terminal extremity of the second domain or to the C-terminal extremity of the first domain and to the N-terminal extremity of second domain; or a direct equivalent thereof.

As it is well known, minor changes in an amino acid sequence such as deletion, addition or substitution of one or several amino acids may lead to an allelic form of the original protein which has identical or substantially identical properties. Thus, by the term "direct equivalents thereof" is meant either any single domain CD25 binding molecule (molecule X)
(i) in which the hypervariable regions CDR1, CDR2 and CDR3 taken as a whole are at least 80% homologous, preferably at least 90% homologous, more preferably at least 95 % homologous to the hypervariable regions as shown in Seq. Id. No. 1 in EP 449,769, and,
(ii) which is capable of inhibiting the binding of Interleukin 2 (IL-2) to its receptor substantially to the same extent as a reference molecule having framework regions identical to those of molecule X but having hypervariable regions CDR1, CDR2 and CDR3 identical to those shown in Seq. Id. No. 1 in EP 449,769;
or any CD25 binding molecule having at least two domains per binding site (molecule X')
(i) in which the hypervariable regions CDR1, CDR2, CDR3, CDR1', CDR2' and CDR3' taken as a whole are at least 80 % homologous, preferably at least 90 % homologous, more preferably at least 95 % homologous to the hypervariable regions as shown in Seq. Id. No. 1 and 2 in EP 449,769, the contents of which is herein incorporated by reference, and
(ii) which is capable of inhibiting the binding of IL-2 to its receptor substantially to the same extent as a reference molecule having framework regions and constant parts identical to molecule X' but having hypervariable regions CDR1, CDR2, CDR3, CDR1', CDR2' and CDR3' identical to those shown in Seq. Id. No. 1 and 2 in EP 449,769.

This last criterion may be conveniently tested in various assays as described in EP 449,769.

A CD25 binding molecule may be a chimeric CD25 antibody, especially a chimeric CD25 antibody comprising at least
a) one heavy chain which comprises a variable domain having an amino acid sequence identical or substantially identical to that shown in Seq. Id. No. 1 in EP 449,769, starting with amino acid at position 1 and ending with amino acid at position 117 and the constant part of a human heavy chain; and
b) one light chain which comprises a variable domain having an amino acid sequence identical or substantially identical to that shown in Seq. Id. No. 2 in EP 449,769, starting with glutamic acid at position 1 and ending with glutamic acid at position 104 and the constant part of a human light chain.

The constant part of a human heavy chain may be of the γ1, γ2, γ3, γ4, µ, α1, α2, δ or ε type, preferably of the γ type, more preferably of the γ1 type, whereas the constant part of a human light chain may be of the κ or λ type (which includes the λ1, λ2 and λ3 subtypes) but is preferably of the κ type. The amino acid sequence of all these constant parts are given in Kabat et al., Sequences of Proteins of Immunological Interest, US Department of Health and Human Services, Public Health Service, NIH..

The CD25 binding molecule of the present invention is basiliximab which is commercially available as SIMULECT^{®} from Novartis AG.
Basiliximab suitable for use in accordance with the present invention may be produced by techniques disclosed in EP 449,769, in particular in Examples 1 to 5 of EP 449,769.

As described in EP-B-449,769, basiliximab has, on the basis of observed activity in e.g. a Mixed Lymphocyte Reaction assay, been found to be useful for preventing or treating graft rejection episodes.

In accordance with the present invention it has now surprisingly been found that basiliximab is effective in the treatment of rheumatoid arthritis or inflammatory or hyperproliferative skin diseases.

Therefore the invention provides the use of a CD25 binding molecule for the preparation of a medicament for the treatment of rheumatoid arthritis or inflammatory or hyperproliferative skin diseases, wherein the CD25 binding molecule is basiliximab, as defined in the appended claims.

For the use in accordance with the invention, the appropriate dosage will, of course, vary depending upon, for example, the particular CD25 binding molecule to be employed, the host, the mode of administration and the severity of the condition being treated and the effects desired. Satisfactory results are generally indicated to be obtained at dosages from about 0.1 mg to about 100 mg. Administration may be in a single dose or in several doses over a period of time as long as may be indicated in relation to the time the disease is clinically evident or prophylactically to suppress further clinical relapse, for example a dose of 10 to 100 mg may be administered with a time-lag of one week to five weeks, e.g. every four weeks, up to a time control or amelioration of the disease is achieved. The CD25 binding molecule is conveniently administered parenterally, e.g. intravenously, for example, into the antecubital or other peripheral vein. An exemplary dosing regimen is intravenous administration of 40 mg every 28 days until control or amelioration of the disease is achieved.

The medicament of the invention may be manufactured in a conventional manner as described, e.g. in EP 449,769.

The basiliximab may be administered as the sole active ingredient or together with other drugs in immunomodulating regimens or other anti-inflammatory agents. For example, the basiliximab may be used in accordance with the invention in combination with cyclosporins, rapamycins or ascomycins, or their immunosuppressive analogs, e.g. cyclosporin A, cyclosporin G, FK-506, rapamycin etc.; corticosteroids e.g. prednisone; cyclophosphamide; azathioprene; methotrexate; gold salts, sulfasalazine, antimalarials, brequinar; leflunomide; mizoribine; mycophenolic acid; mycophenolate mofetil; 15-deoxyspergualine; other immuno-suppressive monoclonal antibodies, e.g. monoclonal antibodies to leukocyte receptors, e.g. MHC, CD2, CD3, CD4, CD7, CD28, B7, CD40, CD45, or CD58 or their ligands; or other immunomodulatory compounds, e.g. CTLA4Ig.

If the basiliximab is co-administered with a further drug substance both may be packaged separately within the same container, with instructions for mixing or concomitant administration. Examples of kits include for example a multi-barrelled syringe or a twin pack containing separate unit dose forms.

The intravenous infusions may be prepared as follows: the lyophylized antibodies are mixed together and dispersed into 100 ml sterile buffered saline containing 4.5% wt. of human albumin. This saline dispersion may be administered to the patients either as an intravenous bolus injection or as an intravenous infusion over a 15 minute period.

Investigations so far indicate that the administration of the basiliximab is free from unacceptable side-effects at the dosage levels employed. Particularly, basiliximab is safe, approved by the Federal Drug Administration (FDA) of the United States and is commercially available.

Utility of basiliximab for the treatment of rheumatoid arthritis is shown in the following clinical examples.

60 patients with active rheumatoid arthritis and having a partial response to methotrexate alone are enrolled. A partial response to methotrexate alone is defined as the presence of at least 6 swollen and 9 tender joints and C-reactive protein level >20 mg/l in patients who have been receiving a stable, maximally tolerated methotrexate dose (not exceeding 20 mg/week) for at least 3 months prior to the screening visit (Week -2).

These 60 patients are randomized to one of two treatment groups; those receiving methotrexate plus basiliximab and those receiving methotrexate plus placebo. The methotrexate dose and route of administration remains stable during the course of the study. The patients are also on stable doses of nonsteroidal anti-inflammatory drugs and prednisone.

### Example 1:

Basiliximab is administered intravenously at a dose of 60 mg on Day 0, 40 mg on Day 28 and 40 mg on Day 56. Patients are evaluated at Weeks 2, 4, 6, 8, 10 and 12 for safety, efficacy and disease outcome.

The primary efficacy outcome measure is the attainment of ACR (American College of Rheumatology) criteria for improvement of rheumatoid arthritis at Week 12. ACR (20) criteria defines improvement as 20% improvement in the number of tender and swollen joints, in addition to 20% improvement in at least three of five variables (degree of disability, HAQ (Health Activity Questionnaire); patient global assessment; physician global assessment; pain and C-reactive protein).

Patients receiving basiliximab show an amelioration of the symptoms as compared to patients receiving placebo.

### Example 2:

Basiliximab is administered intravenously at a dose of 0.02 mg on Day 0, 0.2 mg on Day 4, 2 mg on Day 8, 20 mg on Day 12, 40 mg on Day 19 and 60 mg on Day 26. Patients are evaluated at Weeks 2, 4, 6, 8, 10 and 12 for safety, efficacy and disease outcome as in Example 1.

Patients receiving basiliximab show an amelioration of the symptoms as compared to patients receiving placebo.

Utility of the CD25 binding molecules in the treatment of inflammatory or hyperproliferative skin diseases, e.g. psoriasis, is shown in the following clinical example.

24 patients (both male and female) between the ages of 18-70 years of age with moderate to severe plaque psoriasis defined as having a regional or total PASI ≥ 10 and affected BSA > 5% enter into the Screening period (Visit 1, Week -2) and are randomized into one of two CD25 binding molecule dose groups (40 mg or 60 mg).

A CD25 binding molecule, e.g. basiliximab is administered at 40 mg or 60 mg (depending on initial randomization), all subsequent 40 mg or 60 mg 15 min iv infusion doses being administered within 48 h of receipt of every blood flow cytometry measurement demonstrating unsaturated peripheral blood lymphocyte IL-2 receptors during a 12 week treatment period. Blood draws for blood flow cytometry measurement occur every 2-weeks at each scheduled patient visit to measure the percent peripheral blood lymphocyte IL-2 receptor saturation. Saturation is defined and reported as a ≥ 95% reduction in CD25 cells with complete IL-2 receptor saturation and unsaturation is defined and reported as ≤ 95% CD25 cells with complete IL-2 receptor saturation.

Each patient is evaluated every 2 weeks for efficacy (PASI, % affected BSA, global and overall psoriasis evaluation) and photographed using standard photographic methods.

Calculation of the PASI: The head (0.1), trunk (0.3), upper limbs (0.2) and lower limbs (0.4) are assessed separately for erythema, infiltration, scaling. The average degree of severity of each symptom in each of the four body parts, is assigned a score of 0-4. The area covered by lesions on each body part is estimated as a percentage of the total area of that particular body part and score from 0-6 is then assigned. This evaluation takes into account the true area affected and not a 'functional' area affected. Further practical details help the assessment: The buttocks count as part of the legs; the axillae and groin count as part of the trunk; the neck counts as part of the head; when scoring the severity of erythema, scales are not removed.

| PASI - Scoring System | | | | | |
|---|---|---|---|---|---|
| Score | 0 | 1 | 2 | 3 | 4 |
| Erythema Infiltration Desquamation | none | slight | moderate | severe | very severe |

| PASI - Scoring System | | | | | | | |
|---|---|---|---|---|---|---|---|
| Score | 0 | 1 | 2 | 3 | 4 | 5 | 6 |
| True Area % | 0% | 1%-9% | 10%-29% | 30%-49% | 50%-69% | 70%-89% | 90%-100% |

The total score for each body part is obtained by multiplying the sum of the severity scores of the 3 basic lesions by the area score, then multiplying the result by the constant weighted value (head = 0.1, trunk = 0.3, upper limbs = 0.2. lower limbs = 0.4) assigned to that body part. The sum of the scores of the individual body parts gives the PASI.

Affected surface determination (rule of 9's): An estimate of the percent BSA affected with psoriasis is estimated. Each section of the body is labeled with the percent of the total BSA it represents.

Overall psoriasis evaluation: An overall evaluation by patient and investigator is performed comparing impression of improvement while on the drug when related to pretreatment condition. The following scale is used: 1 = very good; 2 = good; 3 = moderate; 4 = slight; 5 = none.

The investigator's and patient's overall psoriasis evaluation are performed at Visit 6 (Week 6), Visit 7 (Week 8), Visit 8 (Week 10), Visit 9 (Week 12), Visit 10 (Week 16), and Visit 11 (Week 20).

Global psoriasis evaluation: A global evaluation of the current status of the disease is rated by the investigator using the following scale: 1 = totally clear; 2 = almost clear; 3 = mild; 4 = mild-moderate; 5 = moderate; 6 = moderate-severe; 7 = severe.

Patients receiving basiliximab show a marked improvement with respect to PASI, Global Psoriasis Evaluation, BSA, and Overall Psoriasis Evaluation as compared to patients receiving placebo.

## Claims

1. Use of a CD25 binding molecule for the preparation of a medicament for the treatment of rheumatoid arthritis or inflammatory or hyperproliferative skin diseases, wherein the CD25 binding molecule is basiliximab.

2. Use according to claim 1 wherein the medicament is combined with at least one pharmaceutical composition comprising a further drug substance effective in the treatment of rheumatoid arthritis or inflammatory or hyperproliferative skin diseases.

3. Use according to claim 1 or claim 2 wherein the CD25 binding molecule is combined with at least one further drug substance effective in the treatment of rheumatoid arthritis or inflammatory or hyperproliferative skin diseases.

4. Use according to any preceding claim wherein the CD25 binding molecule is combined with at least one of cyclosporins, rapamycins, ascomycins, or their immunosuppressive analogues, corticosteroids, immuno-suppressive monoclonal antibodies, monoclonal antibodies to leucocyte receptors or immunomodulating compounds.

5. Use according to claim 4 wherein the CD25 binding molecule is combined with at least one of cyclosporin A, cyclosporine G, FK-506, prednisone, cyclophosphamide, azathioprene, methotrexate, gold salts, sulfasalazine, antimalarials, brequinar, leflunomide, mizoribine, mycophenolic acid, mycophenolate mofetil, 15-deoxyspergualine, monoclonal antibodies to MHC, CD2, CD3, CD4, CD7, CD28, B7, CD40, CD45 or CD58 receptors, or CTLA4Ig.

6. Use according to any preceding claim wherein the medicament is administered with a non-drug inflammatory or hyperproliferative skin disease therapy.

7. Use according to claim 6 wherein the non-drug therapy is UV light therapy.

8. Use according to any preceding claim wherein the inflammatory or hyperproliferative skin disease is psoriasis.

## Patentansprüche

1. Verwendung eines CD25 Bindemoleküls zur Herstellung eines Arzneimittels für die Behandlung von rheumatoider Arthritis oder inflammatorischer oder hyperproliferativer Hautkrankheiten, worin das CD25 Bindemolekül Basiliximab ist.

2. Verwendung nach Anspruch 1, worin das Arzneimittel kombiniert ist mit wenigstens einer pharmazeutischen Zusammensetzung, die einen weiteren Wirkstoff umfasst, der für die Behandlung von rheumatoider Arthritis oder inflammatorischer oder hyperproliferativer Hautkrankheiten wirksam ist.

3. Verwendung nach Anspruch 1 oder 2, worin das CD25 Bindemolekül kombiniert ist mit wenigstens einem weiteren Wirkstoff, der für die Behandlung von rheumatoider Arthritis oder inflammatorischer oder hyperproliferativer Hautkrankheiten wirksam ist.

4. Verwendung nach einem der vorhergehenden Ansprüche, worin das CD25 Bindemolekül kombiniert ist mit wenigstens einem Cyclosporin, Rapamycin, Ascomycin oder einem immunsuppressiven Analogon hiervon, einem Corticosteroid, immunsuppressiven monoklonalen Antikörper, monoklonalen Antikörper für Leukozytenrezeptoren oder einer immunmodulierenden Verbindung.

5. Verwendung nach Anspruch 4, worin das CD25 Bindemolekül kombiniert ist mit wenigstens einem Cyclosporin A, Cyclosporin G, FK-506, Prednison, Cyclophosphamid, Azathiopren, Methotrexat, Goldsalz, Sulfasalazin, Antimalariamittel, Brequinar, Leflunomid, Mizoribin, Mycophenolsäure, Mycophenolatmofetil, 15-Desoxyspergualin, monoklonalen Antikörper als Rezeptor für MCH, CD2, CD3, CD4, CD7, CD28, B7, CD40, CD45 oder CD58, oder CTLA41g.

6. Verwendung nach einem der vorhergehenden Ansprüche, worin das Arzneimittel verabreicht wird mit einer Nichtarzneimittel-Therapie (Nondrug) einer inflammatorischen oder hyperproliferativen Hautkrankheit.

7. Verwendung nach Anspruch 6, worin die Nichtarzneimittel-Therapie (Nondrug) eine Therapie mit UV Licht ist.

8. Verwendung nach einem der vorhergehenden Ansprüche, worin die inflammatorische oder hyperproliferative Hautkrankheit Psoriasis ist.

## Revendications

1. Utilisation d'une molécule de liaison à CD25 pour la préparation d'un médicament pour le traitement de la polyarthrite rhumatoïde ou de maladies cutanées inflammatoires ou hyperprolifératrices, dans laquelle la molécule de liaison à CD25 est le basiliximab.

2. Utilisation selon la revendication 1 dans laquelle le médicament est combiné avec au moins une composition pharmaceutique comprenant une autre substance médicamenteuse efficace dans le traitement de la polyarthrite rhumatoïde ou de maladies cutanées inflammatoires ou hyperprolifératrices.

3. Utilisation selon la revendication 1 ou la revendication 2 dans laquelle la molécule de liaison à CD25 est combinée avec au moins une autre substance médicamenteuse efficace dans le traitement de la polyarthrite rhumatoïde ou de maladies cutanées inflammatoires ou hyperprolifératrices.

4. Utilisation selon l'une quelconque des revendications précédentes dans laquelle la molécule de liaison à CD25 est combinée avec au moins une molécule parmi les cyclosporines, les rapamycines, les ascomycines ou leurs analogues immunosuppresseurs, les corticostéroïdes, les anticorps monoclonaux immunosuppresseurs, les anticorps monoclonaux dirigés contres des récepteurs de leucocyte ou des composés immunomodulateurs.

5. Utilisation selon la revendication 4 dans laquelle la molécule de liaison à CD25 est combinée avec au moins une substance parmi la cyclosporine A, la cyclosporine G, FK-506, le prednisone, le cyclophosphamide, l'azathioprène, le méthotrexate, des sels d'or, la sulfasalazine, des antipaludiques, le bréquinar, le leflunomide, la mizoribine, l'acide mycophénolique, le mycophénolate mofétil, la 15-désoxyspergualine, des anticorps monoclonaux dirigés contres les récepteurs CMH, CD2, CD3, CD4, CD7, CD28, B7, CD40, CD45 ou CD58 ou CTLA4Ig.

6. Utilisation selon l'une quelconque des revendications précédentes dans laquelle le médicament est administré avec un traitement non médicamenteux d'une maladie cutanée inflammatoire ou hyperprolifératrice.

7. Utilisation selon la revendication 6 dans laquelle le traitement non médicamenteux est un traitement à la lumière UV.

8. Utilisation selon l'une quelconque des revendications précédentes dans laquelle la maladie cutanée inflammatoire ou hyperprolifératrice est le psoriasis.
